# EUROPEAN PATENT APPLICATION

(11) **EP 1 958 611 A1**
(43) Date of publication of application: **20.08.2008**
(21) Application number: 06026377.9
(22) Date of filing: 20.12.2006
(51) Int. Cl.: A61K 8/31, A61K 8/49, A61Q 19/08

(54) **Oral composition containing EGCG and lycopene**

(71) Applicant: DSM IP Assets B.V., 6411 TE Heerlen (NL)
(72) Inventor: The designation of the inventor has not yet been filed
(74) Representative: Rabanus, Birgit

(57) **Abstract**

Composition containing the combination of (-)-epigallocatechin gallate (EGCG) and lycopene characterized in that the ratio of EGCG to lycopene is in the range of 100 : 1 to 1 : 1.

## Description

The present invention relates to an oral composition containing (-)-epigallocatechin gallate (EGCG) and lycopene in a specific ratio in order to prevent sunlight-induced aging (photoaging) of the skin.

If the skin is chronically exposed to UV radiation this leads to epidermal and dermal damage, such as hyperkeratosis, keratinocyte dysplasia and dermal elastosis in the affected skin areas, clinically presenting as photoaged skin with actinic or solar keratosis. The molecular mechanisms of skin photodamage and photoaging have been subject of extensive research. UV radiation activates a whole range of cell surface growth factors and cytokine receptors (Rittie and Fisher 2002). This ligand-independent receptor activation induces multiple downstream signalling pathways that converge to stimulate the transcription factor AP-1. Among the genes that are up-regulated by AP-1 are several matrix metalloprotease (MMP) family members.

MMPs are therefore amongst other things responsible for solar UV radiation-induced skin damage, affecting skin tone and resiliency leading to premature aging. MMPS degrade collagen and elastin in the extracellular matrix of skin. Increased MMP expression and activity causes enhanced collagen proteolysis, and together with reduced collagen expression results in skin elastosis and wrinkling (Berneburg 2003). The symptoms of that include leathery texture, wrinkles, mottled pigmentation, laxity and sallowness.

MMPs are among the most important and well established photoaging associated genes. To establish the protective abilities of dietary ingredients with regard to photoaging the investigation of their effect on suppression of UVA-induced MMPs is therefore of major interest. (-)-epigallocatechin gallate (EGCG) is among the dietary compounds which suppress various skin collagenases (Lee et al., 2005). Also betacarotene - a pro-vitamin A carotenoid - has been found to repress UVA-induced collagenases (Wertz et al., 2004).
On the other hand a non-pro-vitamin A dietary carotenoid - lycopene - has been described to further increase UVA-induced MMP1 (Offord et al., 2002). This increase was only prevented in combination with vitamin E.

It was therefore an object of the present invention to provide active ingredients that prevent sunlight-induced aging (photoaging) of the skin.

It was therefore an object of the present invention to provide active ingredients that prevent sunlight-induced aging (photoaging) of the skin.

It has surprisingly been found that the object of the present invention is achieved by a synergistic combination of EGCG with lycopene, especially by an oral composition containing a combination of EGCG and lycopene in a ratio in the range of 100 : 1 to 1 : 1.

It was not to be foreseen by the person skilled in the art that the combination of EGCG with lycopene would demonstrate superior synergistic activity in suppressing UVA-induced MMP1 and thus provide an antiaging/anti-wrinkling effect. The synergistic combination of lycopene and EGCG also prevents photoaging when given days to weeks before actual sun/UV-light exposure. It also protects when taken shortly before during and after intensive sun exposure, i.e. sun bathing.

The term "EGCG" as used herein denotes (-)-epigallocatechin gallate and/or one or more of its derivatives (e. g. esterified forms, glycosides, sulphates) thereof. Especially preferred is (-)-epigallocatechin gallate itself.

In preferred embodiments of the present invention the used EGCG has a purity of at least 80 %, preferably of at least 85 %, more preferably of at least 90 %, even more preferably of at least 92 %, most preferably of at least 94 %.
Especially preferred is also an aqueous green tea extract containing EGCG in an amount of at least 80 % (preferred of at least 85 %, more preferred of at least 90 %, even more preferred of at least 92 %, most preferred of at least 94 %), based on the total amount of the extract, as e.g. and preferably obtained by any of the processes described in US 6,383,392, EP 1 103 550, US 10/246 112 and EP 1 077 211. Preferably the total amount of other polyphenols and catechins such as gallocatechin gallate, catechin gallate, epicatechin gallate, epigallocatechin, gallocatechin and epicatechin is less than or equal to 5 % by weight, based on the total weight of the green tea extract. More preferably the amount of gallocatechin gallate is less than or equal to 2.5 % by weight, and/or the amount of epicatechin gallate is less than or equal to 5 % by weight (preferably less than or equal to 3 % by weight).
According to the present invention it is advantageous if the amount of caffeine in the green tea extract is less than or equal to 2.5 % by weight, preferably less than or equal to 0.1 % by weight, and/or the amount of gallic acid is less than or equal to 0.1 % by weight, each based on the total weight of the green tea extract.

The term "lycopene" as used herein includes all-E and Z-stereoisomers. Alternatively a tomato extract which contains high amounts of lycopene can also be used.

According to the present invention it is advantageous if the ratio of EGCG to lycopene is in the range of 100 : 1 to 1 : 1, preferred in the range of 50 : 1 to 3 : 1, most preferred in the range of 25 : 1 to 5 : 1.

According to the present invention it is advantageous to administer the active ingredients in a way that their effective daily amounts ("daily dosages") are in the ranges given below. It is thereby irrelevant if the daily dosage is applied all at once (by a single dosage) or in multiple dosages.

EGCG: daily dosage for humans with a body weight of about 70 kg: 50 to 600 mg, preferred daily dosage for humans with a body weight of about 70 kg: 150 to 300 mg.

Lycopene: daily dosage for humans with a body weight of about 70 kg should not exceed 40 mg, preferably not exceed 25 mg; the daily dosage for humans with a body weight of about 70 kg is advantageously between 0.1 to 40 mg, more preferably between 0.5 to 25 mg.

If the composition is prepared in form of tablets, capsules, granules or powder for oral administration, there may be used excipients such as lactose, sucrose, sodium chloride, glucose, urea, starch, dextrins and/or maltodextrins, calcium carbonate, calcium phosphate and/or calcium hydrogen phosphate, kaolin, crystalline and/or microcrystalline cellulose and/or silicic acid as carriers; binders such as water, ethanol, propanol, simple syrup, glucose solution, starch and/or hydrolyzed starch solution, gelatin solution, carboxymethylcellulose, hydroxypropylcellulose, hydroxypropylstarch, shellac, methylcellulose, ethylcellulose, calcium phosphate and/or polyvinyl pyrrolidone; disintegrators such as dry starch, croscarmellose, crospovidone, sodium alginate, agar powder, laminaran powder, sodium hydrogencarbonate, calcium carbonate, polyoxyethylene sorbitan fatty acid esters, sodium lauryl sulfate, stearic acid monoglyceride, starch and/or lactose; disintegration-preventing agents such as stearic acid, cacao butter and/or hydrogenated oils; absorbefacients such as quaternary ammonium bases and/or sodium lauryl sulfate; humectants such as glycerol and/or starch; adsorbents such as starch, lactose, kaolin, bentonite and/or colloidal silica; lubricants such as purified talc, stearic acid salts, boric acid powder and/or polyethylene glycol; taste corrigents such as sucrose, orange peel, citric acid and/or succinic acid; and the like.

If the composition is prepared in the form of tablets, these may be provided as tablets coated with usual coatings, for example, sugar-coated tablets, gelatin-coated tablets, enteric coated tablets, film-coated tablets, double coated tablets, multilayer-coated tablets and the like. The capsules are prepared by mixing the compounds according to the present invention with the various carriers exemplified above or according to the current state of the art and charging the mixture into hard gelatin capsules, soft capsules and the like.

A multi-vitamin and mineral supplement may be added to the compositions according to the present invention, e.g. to maintain a good balanced nutrition or to obtain an adequate amount of an essential nutrient missing in some diets. The multi-vitamin and mineral supplement may also be useful for disease prevention and protection against nutritional losses and deficiencies due to lifestyle patterns and common inadequate dietary patterns sometimes observed in diabetes.

The composition according to the present invention can be a food or beverage composition. Beverages can be e.g. sports drinks, energy drinks or other soft drinks, or any other suitable beverage preparation.

A sports drink is a beverage which is supposed to rehydrate athletes, as well as restoring electrolytes, sugar and other nutrients. Sports drinks are usually isotonic, meaning they contain the same proportions of nutrients as found in the human body.

Energy drinks are beverages which contain (legal) stimulants, vitamins (especially B vitamins) and/or minerals with the intent to give the user a burst of energy. Common ingredients include caffeine, guarana (caffeine from the Guarana plant) and/or taurine, various forms of ginseng, maltodextrin, inositol, carnitine, creatine, glucuronolactone, coenzyme Q10 and/or ginkgo biloba. Some may contain high levels of sugar, or glucose, whereas others are sweetened completely or partially with a sugar alcohol and/or an artificial sweetener like Ca-cyclamate or Aspartame. Many such beverages are flavored and/or colored.

A soft drink is a drink that does not contain alcohol. In general, the term is used only for cold beverages. Hot chocolate, tea, and coffee are not considered soft drinks. The term originally referred exclusively to carbonated drinks, and is still commonly used in this manner.

If the composition is prepared in form of one of the following food articles it is according to the present invention advantageous if the amount of EGCG and the amount of lycopene in the composition are selected from the ranges given in the following table:

| **Food Category** | **typical serving size** | **mg EGCG per L beverage or kg food** | **mg lycopene per L beverage or kg food** | **Ratio (EGCG : lycopene)** |
|---|---|---|---|---|
| Beverages (final product) (e.g. soft drinks, juices, teas, soups) | 250 mL | 100 to 600, preferred 200 to 400 | 4 to 20 | 10 : 1 to 50 : 1 |
| Dairy Products (e.g. milk shakes, joghurts, ice creams) | 150 g | 170 to 1'000 | 4 to 20 | 10 : 1 to 50 : 1 |
| Sweets (e.g. chocolates, candies, mints, jellyies) | 1 to 5 pieces of 5 g each per day = 5 to 25 g | 500 to 10'000, preferred 500 to 1'000 | 50 to 200 | 10 : 1 to 100: 1 |
| other food items (e.g. cookies, muesli bars) | 25 g | 400 to 4'000, preferred 400 to 1'400 | 40 to 200 | 10 : 1 to 50 : 1 |

If the composition is prepared in form of a tablet or a capsule it is according to the present invention advantageous if the amount of EGCG and the amount of Lycopene in the composition are selected from the ranges given in the following table:

| **Category** | **typical dosage** | **mg EGCG per tablet/capsule** | **mg lycopene per tablet/capsule** | **Ratio (EGCG : lycopene)** |
|---|---|---|---|---|
| Tablets | 1-2 x per day | 50 to 150 | 1 to 10 | 5 : 1 to 100 : 1 |
| Capsules | 1-2 x per day | 50 to 150 | 1 to 10 | 5 : 1 to 100: 1 |

### Examples:

### Example 1:

### Synergistic effects on inhibition of UV-A induced expression of matrix metalloproteinase-1

Primary human dermal fibroblasts were cultured in EMEM without glutamine (Earle's Minimum Essential Medium), supplemented with antibiotics / antimycotics, 2 mM L-glutamine and 7.5 % fetal calf serum at 37 °C/5 % CO₂ and grown to 100 % confluency. Forty-eight hours prior to irradiation the cells were preincubated with the desired substances in EMEM (with AB/AM, L-Glutamine and 2 % FCS. After 24 hours the medium was replaced by fresh medium with substances (fresh prepared). For irradiation, the medium was removed and after 6 wash steps with phosphate-buffered saline (PBS) replaced by phosphate-buffered saline (PBS). The plate was then exposed to 30 J/cm² UVA1. The UVA1 output was approximately 150 mW/cm². After irradiation the phosphate-buffered saline in the cell microplate was exchanged against culture medium (+ 7.5 % FCS) with the substances (fresh prepared) and the cells were incubated in a CO₂-incubato for further 24 hrs (MMP-1 determination). The culture medium was removed, the cells were rinsed with phosphate-buffered saline and the whole plate was frozen in liquid nitrogen. Total RNA was isolated using RNeasy Total RNA Kits (Qiagen, Hilden; Germany). The RNA concentration was determined via photometric measurement at 260/280. Aliquots of total RNA (75 ng) were applied for cDNA-Synthesis using Superscript^{™}III First-Strand synthesis system for RT-PCR. Two samples for each compound were analyzed. The PCR reactions were carried out on an Opticon 1 (MJ Research, Waltham, MA, USA) using SYBR Green^{®} PCR Master Mix (Applied Biosystems, Darmstadt, Germany). For comparison of relative expression in real time PCR control cells and treated cells the 2^{(-delta delta C(T))} method was used.

### Results are summarized in Table 1.

UV-A treatment induced an ~10-fold increase of MMP-1 RNA compared to the levels detected in non-irradiated cells (not shown). EGCG and lycopene reduced this expression by 13 % and 65 %, respectively. In contrast, when the substances were combined, UV-A induced MMP-1 expression was completely abolished. This means that EGCG and lycopene exert a synergistic effect on the modulation of MMP-1, since a positive value (*i.e.* 22 %) was obtained between the observed and expected inhibition (sum of the values of each single compound).

**Table 1: Expression of MMP-1 in skin fibroblasts**

| *treatment* | *compound* | *MMP-1 expression relative to UV-A only treated cells* | % *inhibition* | Δ *(observed-expected inhibition)* |
|---|---|---|---|---|
| UV-A | - | (100 %) | - | - |
| UV-A | EGCG | **87** | **13** | - |
| UV-A | Lycopene | **35** | **65** | - |
| UV-A | EGCG + Lycopene | **0** | **100** | **22** |

### Example 2:

Tablet for long-term anti-aging prevention

**Composition:**

| | **Potency/tabl.** | **Amount/tabl.** |
|---|---|---|
| EGCG (as TEAVIGO^{™}TG) | 300 mg | 333 mg |
| Lycopene (as redivivo^{™} 10% CWS/S-TG) | 10 mg | 100mg |
| Agglomerated lactose (Tablettose ^{™} 80) | | 125 mg |
| Microcrystalline cellulose (Tablettose ^{™} 80) | | 312 mg |
| Silicon dioxide (Aerosil ^{™} 200) | | 5 mg |
| Crospovidone NF (as Polyplasdone ^{™} XL 10) | | 20 mg |
| Magnesium stearate | | 5 mg |
| Tablet weight | | 900 mg |

### Preparation:

Redivivo^{™} (Lycopene) 10% CWS/S-TG, TEAVIGO^{™} TG, agglomerated lactose, microcrystalline cellulose, silicon dioxide and Crospovidone are added to an appropriate vessel and mixed for 20 minutes by a tumbler mixer. Magnesium stearate is sieved through a 1 mm sieve, added and the composition again mixed for 2 min.
The powder is compressed to tablets with a Korsch XP1 tablet press, punch size 17x7.87 mm oblong.

One tablet per day should be taken starting in spring, i.e. at least two months before increased sun exposure, and maintained throughout season.

### Example 3:

Instant drink for boosting anti-aging protection

**Composition:**

| | |
|---|---|
| | **Parts** |
| Xylitol | 663.4 |
| Citric acid, anhydrous | 220.0 |
| Carboxymethyl cellulose | 30.0 |
| Tri-sodium-citrate | 22.0 |
| Tri-calcium-phosphate | 20.0 |
| Orange flavour | 20.0 |
| redivivo^{™} 10% CWS/S-TG | 6.0 |
| Ascorbic acid | 8.0 |
| Sweetener Twinsweet | 4.0 |
| EGCG as TEAVIGO^{™} | 6.6 |
| Powder mix total | 1000.0 |

### Preparation:

Sieve all ingredients through a 500 µm sieve.
Give the powder in an appropriate container and mix on a tumbler blender for at least 20 min. Use 35 g powder for one litre of beverage by adding water.

The instant drink contains 50 mg EGCG and 5 mg Lycopene per serving of 240 ml ready drink.

Up to 3 servings per day during and after extensive or sun bathing is recommended.

### Example 4

Mints for continuous basic anti-aging protection:

**Composition:**

| | Potency/mint | Amount/mint |
|---|---|---|
| EGCG (as TEAVIGO^{™} TG) | 10 mg | 11.1 mg |
| Lycopene (as redivivo^{™} 10% CWS/S-TG) | 0.5 mg | 5 mg |
| Vitamin C as Ascorbic Acid fine granular | | 10.5 mg |
| Sorbitol as Neosorb 60 W | | 163.6 mg |
| Silicon dioxide (Aerosil ^{™} 200) | | 1 mg |
| Aroma Frescoforte Permaseal 60470-31 (Givaudan) | | 10 mg |
| Aroma Eiszucker Permaseal 60153-73 (Givaudan) | | 6.0 mg |
| Sweetener as Twinsweet | | 1.6 mg |
| PEG 6000 | | 20.0 mg |
| Magnesium stearate | | 1.2 mg |
| Total Weight | | 230.0 mg |

### Preparation:

Mix TEAVIGO^{™} TG, redivivo^{™} 10% CWS/S-TG, Ascorbic Acid fine granular, Sorbitol, the aromas, sweetener and PEG 6000 into a drum and mix for 10 minutes with a tumbler mixer. Sieve Sorbitol and silicon dioxide through a 1 mm sieve and mix it in a separate drum for 10 minutes. Combine the two powder mixtures and mix again for 10 minutes. Add Magnesium stearate after sieving through a 1 mm sieve and mix for 2 minutes.

The powder is compressed to tablets with a Korsch XP1 tablet press, punch size 8mm round.
Up to 5 mints per day are recommended.
TEAVIGO^{™}, TEAVIGO^{™}TG and redivivo^{™} 10% CWS/S-TG: Tradeproducts of DSM Nutritional
Products;
Tablettose ^{™} 80: Tradeproduct of Brenntag N.V.;
Aerosil ^{™} 200: Tradeproduct of Degussa;
Polyplasdone ^{™} XL 10: Tradeproduct of ISP.

## Claims

1. Composition containing the combination of (-)-epigallocatechin gallate (EGCG) and lycopene **characterized in that** the ratio of EGCG to lycopene is in the range of 100 : 1 to 1 : 1.

2. Composition according to claim 1 **characterized in that** the ratio of EGCG to lycopene is in the range of 50 : 1 to 3 : 1.

3. Composition according to claim 1 **characterized in that** the ratio of EGCG to lycopene is in the range of 25 : 1 to 5 : 1.

4. Composition according to any of claims 1 to 3 **characterized in that** the composition is suitable for oral consumption.

5. Composition according to claim 4 **characterized in that** the composition is a beverage.

6. Composition according to claim 4 **characterized in that** the composition is a dairy product.

7. Composition according to claim 4 **characterized in that** the composition is a sweet.

8. Use of a composition according to any of claims 1 to 7 for suppression of sun-light induced collagenases.

9. Use of a composition according to any of claims 1 to 7 for reduction of sun-light induced collagen and elastin degradation.

10. Use of a composition according to any of claims 1 to 7 for prevention and/or reduction of sun-light induced wrinkling and skin aging.
